# EUROPEAN PATENT APPLICATION

(11) **EP 1 975 179 A1**
(43) Date of publication of application: **01.10.2008**
(21) Application number: 08158615.8
(22) Date of filing: 13.08.2003
(51) Int. Cl.: C07K 16/18, G01N 33/68

(54) **Polyclonal antibodies, preparation method thereof and use of same**

(30) Priority: 12.09.2002 ES 202000094
(62) Divisional of application: 03795029.2
(71) Applicant: Araclon Biotech, S.L., 50005 Zaragoza (ES)
(72) Inventor: Sarasa Barrio, Manuel, 50005, Zaragoza (ES)
(74) Representative: Pons Arino, Angel

(57) **Abstract**

Polyclonal antibodies, preparation method thereof and use of same.

The invention relates to polyclonal antibodies which specifically recognize, and have a strong affinity with, the two most significant amyloid peptides, Aβ40 and Aβ42. The invention also relates to the use thereof in assessing drugs which activate the degradation of amyloid peptides characteristic of Alzheimer's disease as well as drugs which inhibit the formation of same.

## Description

The present invention relates to polyclonal antibodies that specifically recognize, and have a strong affinity with, the two most significant amyloid peptides, Aβ40 and Aβ42, as well as the use thereof in assessing drugs which activate the degradation of amyloid peptides characteristic of Alzheimer's disease as well as drugs which inhibit the formation of same. Similarly, they can be useful for assessing the activity of the enzymes implicated in processing the precursor protein of the aforementioned peptides or the activity of the enzymes implicated in the degradation thereof, as well as for assaying the level of expression of genes implicated in the whole machinery of events that leads to the deposition and formation of amyloid plaques, characteristic brain lesions in patients who suffer from Alzheimer's disease.

### STATE OF THE ART

Certain factors implicated in Alzheimer's disease concerning biochemical and metabolic phenomena are known. Two morphological and histopathological changes observed in the brains of patients with Alzheimer's disease are neurofibrillar tangles and amyloid deposits.

Intraneuronal neurofibrillar tangles are also present in other degenerative diseases, but the presence of amyloid deposits both in interneuronal spaces (neuritic plaques) and in the surrounding microvasculature (vascular plaques) seems to be a characteristic of Alzheimer's disease. Of these, the neuritic plaques seem to be the most characteristic (Price, D.L. and coworkers, Drug Development Research (1985) 5:59-68).

The main component of these amyloid plaques is a peptide of 40-42 amino acids denominated amyloid peptide Aβ4.

The amyloid peptide Aβ4 is a polypeptide originating from proteolysis of membrane glycoproteins denominated amyloid peptide Aβ4 precursor proteins (βAPP). These precursor proteins comprise 695 to 770 amino acids, all of which are produced by the same gene.

Two main variants of amyloid peptide Aβ4 have been identified, peptide Aβ40 and peptide Aβ42, of 40 and 42 amino acids, respectively, whose tissue distribution is different both in physiological and pathological states.

We have cloned and sequenced the βAPP gene in chicken and we have shown that it is practically identical to the human gene, as it produces highly homologous βAPPs, that is, homologous of the order of 95%, with those of humans, and the peptide Aβ4, characteristic of Alzheimer's disease, is identical to the human peptide. Moreover, the chicken embryo processes βAPPs in such a way that it produces the peptide Aβ4, due to the action of proteolytic enzymes that cause proteolysis of βAPPs at a key site to produce Aβ4, the proteolytic enzyme responsible for splicing the βAPPs to produce Aβ4 is denominated β-secretase.

### DESCRIPTION OF THE INVENTION

The present invention provides polyclonal antibodies, able to specifically recognize by any conventional immunological technique (Western blot, immunohistochemistry, immunoprecipitation, ELISA, RIA, ...) the presence of amyloid peptides Aβ40 and Aβ42, obtained by immunization of mammals, preferably rabbits, with a protein conjugated with a peptide selected from a group that comprises SEQ ID NO: 1, SEQ ID NO: 2, SEQ ID NO: 3, SEQ ID NO: 4, optionally shortened by elimination of the amino acid residues from the N-terminal and/or C-terminal ends, and optionally lengthened by addition of the amino acid residues appropriate for conjugating the protein.

In a particular embodiment of the invention, the peptide corresponds to SEQ ID NO: 1, optionally lengthened by addition of amino acid residues appropriate for conjugating the protein. In another particular embodiment of the invention, the peptide corresponds to SEQ ID NO: 2, optionally lengthened by addition of amino acid residues appropriate for conjugating the protein. In another particular embodiment of the invention, the peptide corresponds to SEQ ID NO: 3, optionally lengthened by addition of amino acid residues appropriate for conjugating the protein. In another particular embodiment of the invention, the peptide corresponds to SEQ ID NO: 4, optionally lengthened by addition of amino acid residues appropriate for conjugating the protein. Although the elimination of terminal amino acid residues does not eliminate the specific activity, the preferred peptides are those of SEQ ID NO: 1, SEQ ID NO: 2, SEQ ID NO: 3 and SEQ ID NO: 4.

The provision of any of the aforementioned peptides, in substantially pure form, is also part of the present invention.

This invention also provides a method for obtaining the aforementioned polyclonal antibodies by immunization of mammals, preferably rabbits, with a protein conjugated to a peptide selected from a group that comprises SEQ ID NO: 1, SEQ ID NO: 2, SEQ ID NO: 3, SEQ ID NO: 4, optionally shortened by elimination of amino acid residues from the N-terminal and/or C-terminal ends, and optionally lengthened by addition of amino acid residues appropriate for protein conjugation.

According to a preferred embodiment of the present invention, the protein used for conjugation with the peptide is keyhole limpet hemocyanin, KHL.

In an even more preferred embodiment of the present invention, the mammals used for immunization with the protein conjugated with the peptide are rabbits.

In accordance with an aspect of the present invention, a new method is provided for assaying drugs which activate the degradation of amyloid peptides characteristic of Alzheimer's disease as well as drugs which inhibit the formation of same, through use of the aforementioned polyclonal antibodies.

Likewise, the method also serves to assay the activity of enzymes (proteases) implicated in processing the precursor protein of the aforementioned peptides or the activity of enzymes implicated in the degradation thereof, through use of the chicken embryo or any of the membranes or extramembranous fluids of embryonic chicken egg, as an animal assay model.

This invention also provides a method for detecting the presence of absence of the amyloid peptides Aβ40 and Aβ42 in a sample, using the chicken embryo or any of the membranes or extramembranous fluids of embryonic chicken egg, as an animal assay model.

According to a preferred embodiment of the present invention, a new method is provided for assessing drugs which activate the degradation of amyloid peptides characteristic of Alzheimer's disease as well as drugs which inhibit the formation of same, through use of chick embryo or any of the membranes or extramembranous fluids of embryonic chicken egg, as an animal assay model.

According to an additional preferred embodiment of the present invention, a new method is provided for assessing the activity of enzymes (proteases) implicated in processing the precursor protein of the aforementioned peptides or the activity of enzymes implicated in the degradation thereof, through use of the chicken embryo or any of the membranes or extramembranous fluids of embryonic chicken egg, as an animal assay model.

The method consists of inoculating the drug in the embryonic chicken egg, either by simple dripping over the embryo itself or one of its membranes, or by injection into the vitellus (if the embryo is young) or vitelline sac (if the embryo is older), into the amniotic sac, into the allantoic sac (in embryos incubated for more than 6 days) or into the embryo itself and, after a suitable incubation time, the embryo and/or any of the membranes or extraembryonic fluids are extracted and the quantity of amyloid peptides, characteristic of Alzheimer's disease, is analyzed using conventional laboratory techniques for quantifying peptides and proteins such as Western blots, immunohistochemistry, immunoprecipitation, ELISA, RIA, HPLC, etc.

### EXAMPLES

The present invention is illustrated with the following examples.

### Example 1

### Coupling of peptides to keyhole limpet hemocyanin (KLH)

The peptides were coupled to keyhole limpet hemocyanin (KLH) by the N-terminal end, using glutaraldehyde as the coupling agent. To do this, KLH protein was activated in borate buffer solution at pH 10. The synthetic peptide was then added and the solution of glutaraldehyde 0.3% was slowly added while stirring at room temperature. After the addition of glycine 1M to block the glutaraldehyde that had not reacted, the peptide-protein conjugate was dialyzed against 3 litres of borate buffer at pH 8.5 and at a temperature of 4°C. The peptide-KLH conjugate was stored at 4°C.

### Example 2

### Generation of polyclonal antibodies

The four polyclonal antibodies were generated by immunization of New Zealand white rabbits against the four peptides coupled to KLH that were used as an immunogen.

Each immunogen was injected into two rabbits, performing five injections, the first an intradermal injection of the peptide-KLH conjugate in PBS and emulsified in complete Freund adjuvant and four more intramuscular injections as a booster dose on Days 14, 28, 49 and 80 of the same peptide-KLH conjugate in PBS but this time emulsified in incomplete Freund adjuvant, performing the control bleeding after 90 days to detect the presence of antibodies.

### Example 3

### Purification of antibodies by affinity

After blood collection, the serum was separated and purified by desalination and then the antibodies were purified by affinity in a matrix comprising 1.5 ml of EMD-epoxy activated material (Merck) to which 5 mg of the corresponding peptide was added. The purified fractions were stabilized in 0.1 % BSA (Sigma) and stored at 4°C. Glycerol 20-50% could be added as a cryoprotection.

### Example 4

### Antibody assay by ELISA

After purification by affinity, the antibody was assayed by ELISA. To do this, the antigen was placed in a Maxi Sorb ELISA plate from Nunc at a concentration of 50 mg/50 µl en PBS pH 7, and the antibody was detected with conjugated mule anti-IgG with alkaline phosphatase, using p-nitrophenyl phosphate (PNPP) as substrate in diethanolamine with 5 mM MgCl₂, pH 9.6 and development after 2 hours.

In conclusion, the antibodies were generated by using the different synthetic peptides described previously coupled to KLH. These synthetic peptides contain a very small number of amino acids, which makes them suitable for production in homogenous antibody chains with predefined epitopes.

### List of sequences.

| | |
|---|---|
| SEQ ID NO: 1 | LVFFAEDV |
| SEQ ID NO: 2 | GLMVGGVV |
| SEQ ID NO: 3 | GLMVGGVVIA |
| SEQ ID NO: 4 | RHDSGYEVHHQK |

In this application, the amino acids are abbreviated using the single-letter codes accepted in the field, as indicated below:

| | | |
|---|---|---|
| A = | Ala = | alanine |
| C = | Cys = | cysteine |
| D = | Asp = | aspartic acid |
| E = | Glu = | glutamic acid |
| F = | Phe = | phenylalanine |
| G = | Gly = | glycine |
| H = | His = | histidine |
| I = | Ile = | isoleucine |
| K = | Lys = | lysine |
| L = | Leu = | leucine |
| M = | Met = | methionine |
| N = | Asn = | asparagine |
| P = | Pro = | proline |
| Q = | Gln = | glutamine |
| R = | Arg = | arginine |
| S = | Ser = | serine |
| T = | Thr = | threonine |
| V = | Val = | valine |
| W | Trp = | tryptophane |
| = | | |
| Y = | Tyr = | tryosine |

The information concerning the identification of the peptide sequences described in the present invention, that accompany the present document in a computer-readable format, is identical to the list of sequences presented accompanying the document.

### NUMBER OF SEQUENCES: 4

INFORMATION FOR SEQUENCE 1:
   SEQUENCE CHARACTERISTICS:
      LENGTH: 8
      TYPE: amino acid
   MOLECULE TYPE: peptide
   SOURCE: chemical synthesis
   SEQUENCE DESCRIPTION
   SEQ ID NO. 1 
Leu Val Phe Phe Ala Glu Asp Val
1               5
INFORMATION FOR SEQUENCE 2:
   SEQUENCE CHARACTERISTICS:
      LENGTH: 8
      TYPE: amino acid
   MOLECULE TYPE: peptide
   SOURCE: chemical synthesis
   SEQUENCE DESCRIPTION
   SEQ ID NO. 2 
Gly Leu Met Val Gly Gly Val Val
1               5
INFORMATION FOR SEQUENCE 3:
   SEQUENCE CHARACTERISTICS:
      LENGTH: 10
      TYPE: amino acid
   MOLECULE TYPE: peptide
   SOURCE: chemical synthesis
   SEQUENCE DESCRIPTION
   SEQ ID NO. 3 
Gly Leu Met Val Gly Gly Val Val Ile Ala
1               5                   10
INFORMATION FOR SEQUENCE 4:
   SEQUENCE CHARACTERISTICS:
      LENGTH: 12
      TYPE: amino acid
   MOLECULE TYPE: peptide
   SOURCE: chemical synthesis
   SEQUENCE DESCRIPTION
   SEQ ID NO. 4

Arg His Asp Ser Gly Tyr Glu Val His His Gln Lys
1               5                   10

## Claims

1. Antibody for the specific recognition of the main forms of the amyloid beta peptide, AB40 and AB42, obtainable by immunization of mammals with a protein conjugated with a peptide selected from the following:
a. the peptide of SEQ ID N° 1, the peptide of SEQ ID N° 2, the peptide of SEQ ID N° 3, the peptide of SEQ ID N° 4:
b. the peptides with a sequence arising from the elimination of the N-terminal and/or C-terminal amino acid residues from SEQ ID N° 1, SEQ ID N° 2, SEQ ID N° 3 or SEQ ID N° 4.
c. and the peptides resulting from adding the amino acid residues necessary for protein conjugation to any of the aforementioned sequences.

2. Antibody according to claim 1, **characterized in that** the protein is Keyhole Limpet Hemocyanin (KLH).

3. Antibody according to anyone of claims 1-2, wherein the antibody is a polyclonal antibody.

4. Substantially pure peptide **characterized in that** it has a sequence of amino acids selected from the group as defined in claim 1.

5. Peptide according to claim 4 **characterized in that** the sequence is selected from SEQ ID N° 1, 2, 3 or 4.

6. Use of a peptide according to anyone of claims 4 or 5 for obtaining the antibodies of anyone of claims 1-3.

7. Method for detecting the presence or absence of the amyloid proteins AB40 and AB42 in a sample, **characterized in that** it comprises bringing said sample into contact with an antibody as defined in claim 1, and detecting the presence or absence of the complex formed by said amyloid proteins and said antibody.

8. Assay method for drugs which activate the degradation of amyloid peptides characteristic of Alzheimer's disease, as well as drugs which inhibit the formation of same, through use of the polyclonal antibodies described in claim 1.


SEQUENCE LISTING

<110>  Araclon Biotech S.L.

<120>  Polyclonal Antibodies, preparation method thereof and use of same

<130>  EP1513.1

<160>  4

<170>  PatentIn version 3.4

<210>  1
<211>  8
<212>  PRT
<213>  Artificial

<220>
<223>  Peptide (chemical synthesis)

<400>  1

Leu Val Phe Phe Ala Glu Asp Val
1               5


<210>  2
<211>  8
<212>  PRT
<213>  Artificial

<220>
<223>  Peptide (chemical synthesis)

<400>  2

Gly Leu Met Val Gly Gly Val Val
1               5


<210>  3
<211>  10
<212>  PRT
<213>  Artificial

<220>
<223>  peptido (chemical synthesis)

<400>  3

Gly Leu Met Val Gly Gly Val Val Ile Ala
1               5                   10


<210>  4
<211>  12
<212>  PRT
<213>  Artificial

<220>
<223>  Peptide (chemical synthesis)

<400>  4

Arg His Asp Ser Gly Tyr Glu Val His His Gln Lys
1               5                   10